# EUROPEAN PATENT APPLICATION

(11) **EP 4 487 852 A1**
(43) Date of publication of application: **08.01.2025**
(21) Application number: 23763702.0
(22) Date of filing: 02.03.2023
(51) Int. Cl.: A61K 31/519, A61K 9/00, A61P 17/14, A61P 15/10, A61M 37/00

(54) **TRANSDERMAL DOSAGE FORM, FOR PROMOTING LOCAL BLOOD FLOW, OF DUAL-ACTING PDE5 INHIBITOR/ORGANIC NITRATE ESTER**

(30) Priority: 02.03.2022 KR 20220026477
(71) Applicant: Yonsei University, University-Industry Foundation(UIF)., Seoul 03722 (KR); Juvic Inc., Seoul 08389 (KR); Topadur Pharma AG, 8952 Schlieren (CH)
(72) Inventor: JUNG, Hyungil, Seoul 03719 (KR); KIM, Youseong, Seoul 04612 (KR); NAEF, Reto, 4310 Rheinfelden (CH); ATZEI, Paola, 87952 Schlieren (CH); FRANCISCO, Rita, 8006 Zurich (CH)
(74) Representative: AWA Sweden AB
(86) International application number: PCT/KR2023/002857
(87) International publication number: WO 2023/167503

(57) **Abstract**

The present invention relates to a transdermal dosage form, for promoting local blood flow, of a dual-acting PDE5 inhibitor/organic nitrate ester, and, more specifically, to: use, for preventing or treating various symptoms or diseases including hair loss, of a compound, or a pharmaceutically acceptable salt thereof, that promotes local blood flow by dual-acting as a soluble guanylate cyclase activator and a phosphodiesterase inhibitor; and a transdermal dosage form thereof. The compound promotes local blood flow so as to exhibit, without side effects, excellent effects of preventing and treating hair loss and other various diseases caused by decreased local blood flow, and, if intradermally injected using a microneedle, exhibits effects, that are remarkably better than those of treatment by application, of preventing and treating hair loss and other various diseases caused by decreased local blood flow even with less treatment frequency, and thus the compound can be effectively used as pharmaceutical and cosmetic compositions for hair loss alleviation and hair growth promotion compositions capable of replacing conventional hair loss therapeutic agents, which had limited application, and also can be used for treating various diseases caused by decreased local blood flow.

## Description

### [Technical Field]

The present invention relates to a dual-mode-of-action PDE5 inhibitor/organic nitrate ester in a transdermal dosage form to increase local blood flow, and more specifically to a use of a compound that acts with dual-pharmacology releasing NO in addition to its PDE 5 inhibition to increase local blood flow or a pharmaceutically acceptable salt thereof for the prevention or treatment of various symptoms or diseases including hair loss, and a transdermal dosage form thereof.

### [Background Art]

Phosphodiesterases (PDEs) are enzymes that catalyzes the hydrolysis and thus the degradation of cyclic adenosine monophosphate (cAMP) and cyclic guanosine monophosphate (cGMP) and thereby regulates intracellular levels of second messengers. Inhibition of PDEs leads to increasing intracellular concentrations of endogenous cAMP/cGMP. Therefore, inhibition of PDE can mediate a variety of physiological mechanisms at different cell and organ levels. Phosphodiesterase type 5 (PDES) hydrolyses cyclic guanylate monophosphate (cGMP) specifically to 5' GMP. The selective inhibition of PDE5 has been validated as a relevant approach and strategies directed to promote inhibition of PDE5 activity have been applied as therapeutic tools, in particular, in neuronal, vascular and cardiovascular diseases and disorders. Moreover, the introduction of PDE5 inhibitors has revolutionized the treatment of male erectile dysfunction (Andersson KE, British Journal of Pharmacology (2018) 175:2554-2565; Das A et al. Pharmacol Ther. (2015) 147:12-21; Dobhal T et al. Critical Review in Pharmaceutical Sciences (2012) 1(3):13-27). Most prominent examples of PDE5 inhibitors are Sildenafil, Tadalafil and Vardenafil, which have been described among others, for example, in WO 99/24433, WO 01/60825, EP 995'751 and WO 2011/075655. Recently a novel class of PDE 5 inhibitors with dual-pharmacology releasing NO in addition to its PDE 5 inhibition in a more than additive fashion has been described in WO/2018/215433.

Meanwhile, hair loss is a symptom of abnormally falling out of a lot of hair. Although there are many cases of hair falling out, abnormal hair loss of beard, eyebrows, public hair, armpit hair and other areas is also referred to as hair loss.

Hair is produced in hair follicles, which are located in the dermis layer above the subcutaneous fat below the epidermal layer. Hair follicles are located on all parts of the body except the lips, palms and soles of the feet, and new hair is made from the hair matrix at the bottom of the hair follicles. The living cells in the hair matrix proliferate and push upward, and when these cells dry quickly and die, they are compressed into a dense and hard mass to form a hair shaft. The hair shaft, which is made of dead protein, is covered with a delicate layer (cuticula pili) composed of platelike scales.

The hair growth cycle consists of three phases: the anagen phase, where hair grows most actively, followed by the catagen phase, where hair degeneration begins, and the telogen phase, where hair growth is stopped. At the end of the telogen phase, the hair falls out and a new cycle begins again as new hair grows from the hair follicles. The anagen phase of eyebrows and eyelashes is about 1 to 6 months, and the anagen phase of hair is about 2 to 6 years, and in general, about 50 to 100 hairs fall out at the end of the telogen phase each day.

The most common type of male hair loss is male pattern baldness or alopecia, which develops gradually over several years and begins most prominently on the crown of the head and progresses to the frontal region. In the case of female hair loss, it occurs in a more dispersed form as the thickness of the hair becomes thinner and often occurs after menopause.

Many studies are being conducted to alleviate or treat alopecia, and in particular, research has been carried out in the cosmetic or pharmaceutical industry for a long time to develop new substances for stimulating hair growth or alleviating hair loss, and accordingly, drug therapy using female hormones, blood flow promoters and the like and hair transplantation have been developed. Currently, the most commonly used drugs for the treatment of hair loss are 2,4-diamino-6-piperidinopyrimidine-3-oxide (also known as 'Minoxidil' formulation, refer to US Patent Nos. 4,193,619 and 4,596,812), which has been approved by the FDA, and finasteride, which is a specific inhibitor of type II 5α-reductase.

Minoxidil formulation is a drug that induces hair growth by increasing blood flow through the vasodilating effect and supplying nutrients to the hair root, and in particular, it is known to be effective for alleviating hair loss symptoms in the forehead area, and pharmaceutical products using the same as an active ingredient are marketed under the trade name Rogaine (trade name of Pharmacia & Upjohn Company). Rogaine is known to reduce hair loss by up to 10% and promote hair growth in men suffering from male pattern baldness, but it has to be applied directly to the scalp from the outside, and it has to be used regularly for a long period of time, and there is a disadvantage that it does not exert a very good effect on hair loss in areas other than the forehead area.

Pharmaceutical products using finasteride as an active ingredient are marketed under the trade name Propecia (trade name of Propecia, Merck & Co., Inc.), and it is known as a pill for oral administration to inhibit hair loss by inhibiting the function of type II 5α-reductase and preventing the conversion of testosterone to dihydrotestosterone (DHT). However, this also requires continuous and regular administration, and for some patients, it has side effects such as decreased libido, erectile dysfunction and the like, and there is a problem that it can be used only for adult men.

Meanwhile, various drug delivery methods have been developed to effectively deliver the aforementioned drugs into the human body and to improve the stability against the external environment, but currently, the Minoxidil formulation has to be applied twice a day, and the Propecia formulation is inconvenient because it has to be taken every day.

Accordingly, as a result of making diligent efforts to develop a novel hair growth agent with improved efficacy compared to conventional hair growth agents, it was found that the compound according to the present invention have shown the excellent therapeutic effects, in particular, as compared to Minoxidil which is one of the most currently used drugs and available in the market. It was also effective for female pattern baldness at a low dose, and also exhibited a hair loss prevention effect. In addition, when the compound according to the present invention was mounted on a microneedle and applied, it was additionally confirmed that it exerted a better effect than the existing hair growth agents only by applying it once every two days, compared to applying the existing hair growth agents twice a day. Moreover, the transdermal administration via shaped dissolving micro needles directly to the primary site of action allows application of efficacious therapeutic doses of the inventive compounds to be significantly lower as compared to Minoxidil, and thus to integrate local efficacy.

In addition, systemic drug exposure with side effects such as systemic arterial hypotension are avoided or very unlikely to occur with the shaped dissolving micro needle transdermal administration compounds and compositions of the invention.

### [Disclosure]

### [Technical Problem]

It is an object of the present invention to provide a novel use of a compound or a pharmaceutically acceptable salt thereof, which increases local blood by dually acting on releasing NO and simultaneously inhibiting phosphodiesterase (PDE5), and to provide an effective administration method thereof.

### [Technical Solution]

In order to achieve the above object, the present invention provides a transdermal pharmaceutical composition comprising a compound of formula **I,** or a pharmaceutically acceptable salt thereof 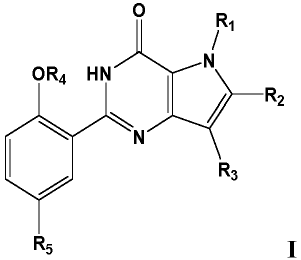 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, said compound of formula I is a compound, wherein R₁ is methyl or ethyl; R₂ is H; R₃ is ethyl or n-propyl; and R₄ is ethyl or n-propyl.

In the present invention, said compound of formula I is a compound, wherein R₁ is ethyl; R₂ is H; R₃ is n-propyl; and R₄ is n-propyl.

In the present invention, said compound of formula **I** is a compound selected from the group consisting of

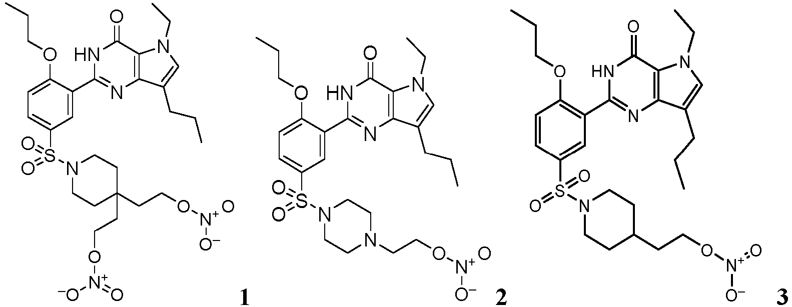

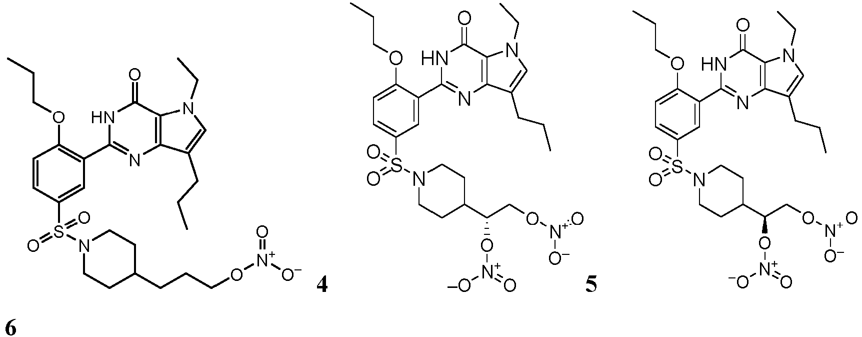

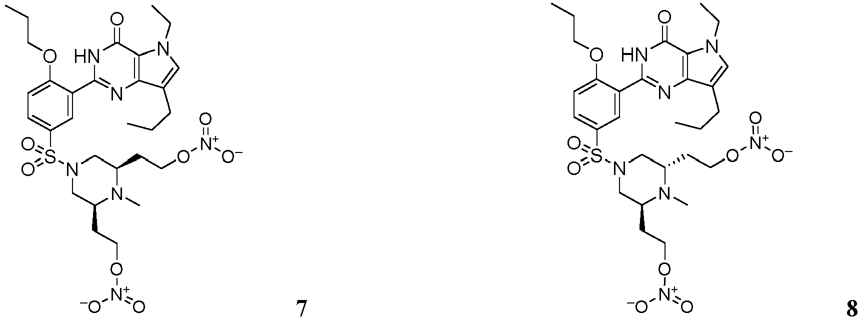

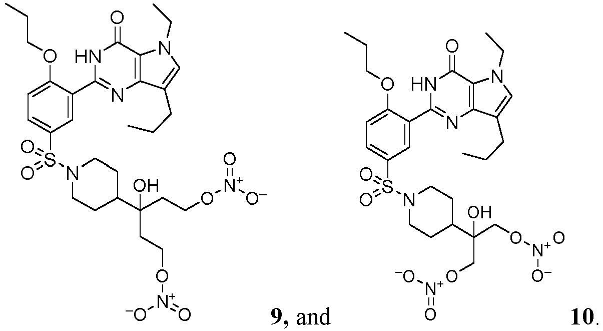

In the present invention, said compound of formula **I** is a compound selected from the group consisting of:
(1-((3-(5-ethyl-4-oxo-7-propyl-4,5-dihydro-3H-pyrrolo[3,2-d]pyrimidin-2-yl)-4-propoxyphenyl)sulfonyl)piperidine-4,4-diyl)bis(ethane-2,1-diyl) dinitrate **(1);**
2-(4-((3-(5-ethyl-4-oxo-7-propyl-4,5-dihydro-3H-pyrrolo[3,2-d]pyrimidin-2-yl)-4-propoxyphenyl)sulfonyl)piperazin-1-yl)ethyl nitrate **(2);**
2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethyl nitrate **(3);**
3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)propyl nitrate **(4);**
(R)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate **(5);**
(S)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate **(6);**
((2R,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate (7);
((2S,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate **(8);**
3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-3-hydroxypentane-1,5-diyl dinitrate **(9);** and
2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-2-hydroxypropane-1,3-diyl dinitrate **(10).**

In a preferred aspect, said compound of formula **I** is Compound 1 (Cp 1) or Compound 2 (Cp 2):

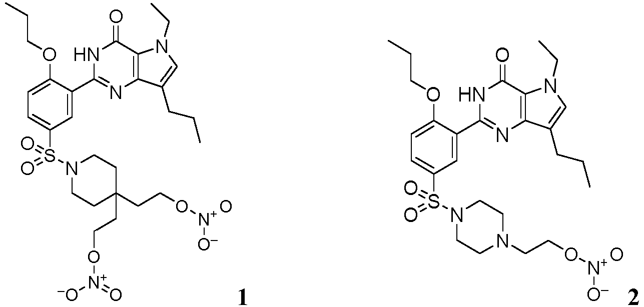

In a very preferred aspect, said compound of formula I is Compound 1.

In the present invention, the transdermal pharmaceutical composition for preventing or treating hair loss may be for topical administration.

In the present invention, the transdermal composition may include 0.00005 to 0.5% (w/w) of the compound represented by Formula **I** preferably said Compound 1 and Compound 2 or the pharmaceutically acceptable salt thereof.

In the present invention, the transdermal composition of Formula I preferably said Compound 1 and Compound 2 above, or the pharmaceutically acceptable salt thereof may be used to prevent, ameliorate, or treat one or more diseases or disorders selected from the group consisting of:
skin diseases, skin aging, steroid-induced skin atrophy;
erectile dysfunction; and
hair loss or hair growth.

In the present invention, the diseases or disorder is hair loss and the pharmaceutical composition may prevent or treat hair loss, or
promote hair growth via
(i) inducing anagen of hair follicles;
(ii) promoting melanogenesis;
(iii) increasing the number of hair follicles;
(iv) increasing skin thickness;
(v) promoting angiogenesis;
(vi) proliferating cells in outer root sheath; and/or
(vii) enhancing blood flow in the hair follicle.

In the present invention, the hair loss may be androgenetic alopecia (AGA), chemotherapy-induced alopecia (CIA) or Alopecia areata.

In another aspect, the present invention provides a compound of formula I, or a pharmaceutically acceptable salt thereof for transdermal use. 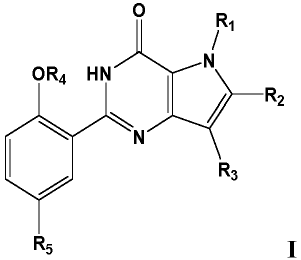 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, the transdermal use is for preventing, ameliorating, or treating one or more diseases or disorders selected from the group consisting of:
skin diseases, skin aging, steroid-induced skin atrophy;
erectile dysfunction; and
hair loss or hair growth.

In another aspect, the present invention provides a use of a compound of formula I, or a pharmaceutically acceptable salt thereof in the manufacture of a transdermal medicine. 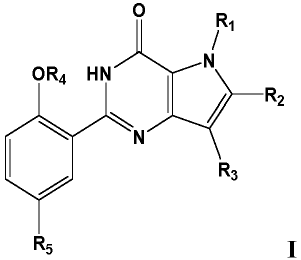 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, the medicine is for use in preventing, ameliorating, or treating one or more diseases or disorders selected from the group consisting of:
skin diseases, skin aging, steroid-induced skin atrophy;
erectile dysfunction; and
hair loss or hair growth.

In another aspect, the present invention provides a transdermal administration method comprising administering a compound of Formula I or a pharmaceutically acceptable salt thereof to a subject in need thereof: 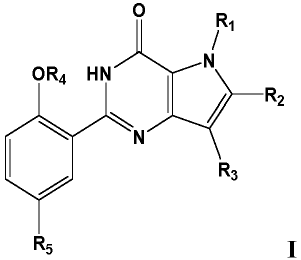 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, the transdermal administration method is for preventing, ameliorating, or treating one or more diseases or disorders selected from the group consisting of:
skin diseases, skin aging, steroid-induced skin atrophy;
erectile dysfunction; and
hair loss or hair growth.

In another aspect, the present invention may be provided a transdermal or topical cosmetic composition comprising a compound of Formula I as an active ingredient.

In addition, the present invention provides a kit comprising:
(i) a first kit component comprising the pharmaceutical composition; and
(ii) a second kit component comprising a patch or device, either adhesive or non-adhesive, suitable for applying the pharmaceutical composition to a skin.

The present invention further provides a solid composition for preparing the pharmaceutical composition, wherein the solid composition is soluble in a liquid solvent, and the liquid composition formed from the dissolved solid composition comprises a compound of Formula I or a pharmaceutically acceptable salt thereof in an amount of 0.00005 to 0.5% (w/w).

In addition, the present invention provides a microneedle structure on which the pharmaceutical or cosmetic composition is mounted.

In the present invention, the microneedle structure may be a microneedle patch or a microneedle applicator.

In the present invention, the microneedle structure may include a solid microneedle, a coated microneedle, a dissolving microneedle, a hollow microneedle or a candlelit-shaped microneedle.

In the present invention, the microneedle structure may comprise a biocompatible polymer selected from the group consisting of hyaluronic acid, collagen, polyvinylpyrrolidone, polylactic acid, polylactic glycolic acid, polylactide, polyglycolic acid, polyethylene glycol, polycaprolactone, poly-amino acid, polypropylene fumarate, poly-gamma glutamic acid, polyvinyl alcohol, polyethyleneimine, albumin, dextran, fibrin, elastin, gelatin, alginic acid, polyacrylic acid, carboxymethylcellulose, and mixtures or copolymers thereof.

In the present invention, the pharmaceutical or cosmetic composition is injected once within 2 to120 15 days using the microneedle structure.

### [Advantageous Effects]

The compound according to the present invention exhibits excellent preventive and therapeutic effects for various diseases caused by reduction of local blood flow such as hair loss without side effects by dually acting as releasing NO and a PDES-phosphodiesterase inhibitor to increase local blood flow, and when it is transdermally injected using a microneedle compared to treatment by application, it exhibits significantly improved preventive and therapeutic effects for various diseases caused by reduction of local blood flow such as hair loss with less treatment frequency. Thus, it can be effectively utilized as transdermal compositions for alleviating hair loss and promoting hair growth, which can replace the existing therapeutic agents for hair loss whose application is limited, and it will be possible to be utilized for the therapeutic use of various diseases caused by reduction in local blood flow.

### [Description of Drawings]

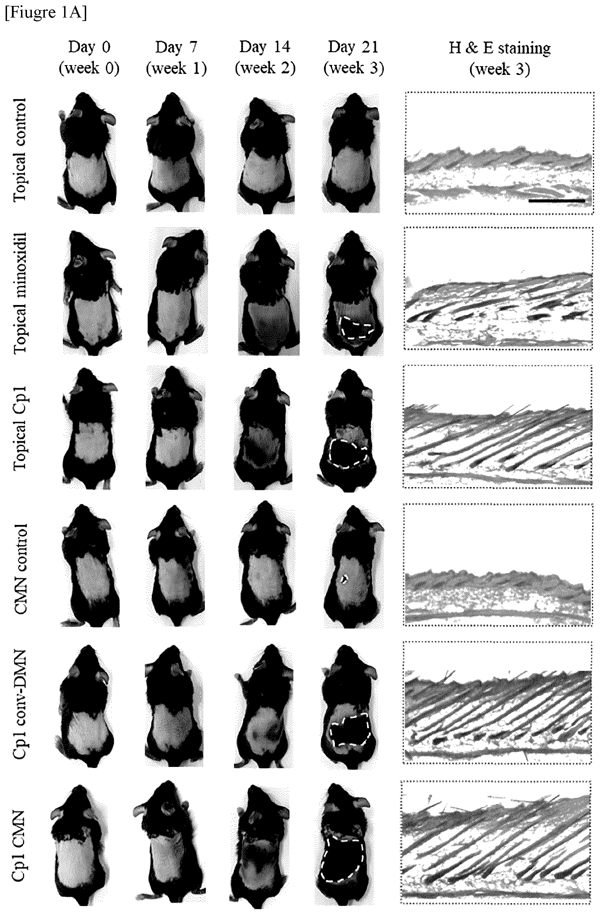

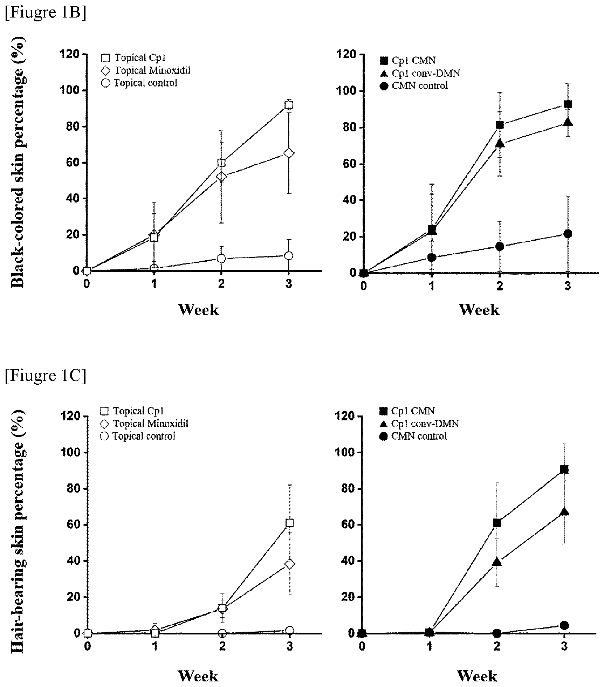

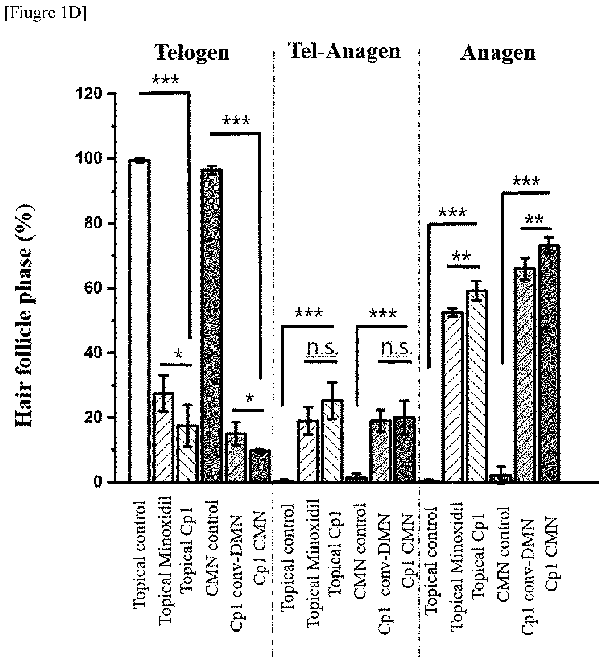

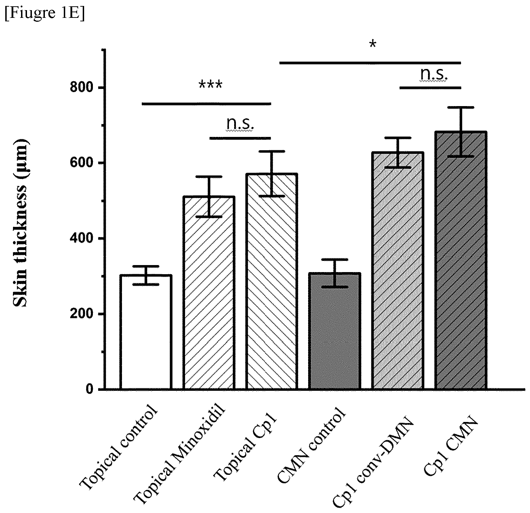

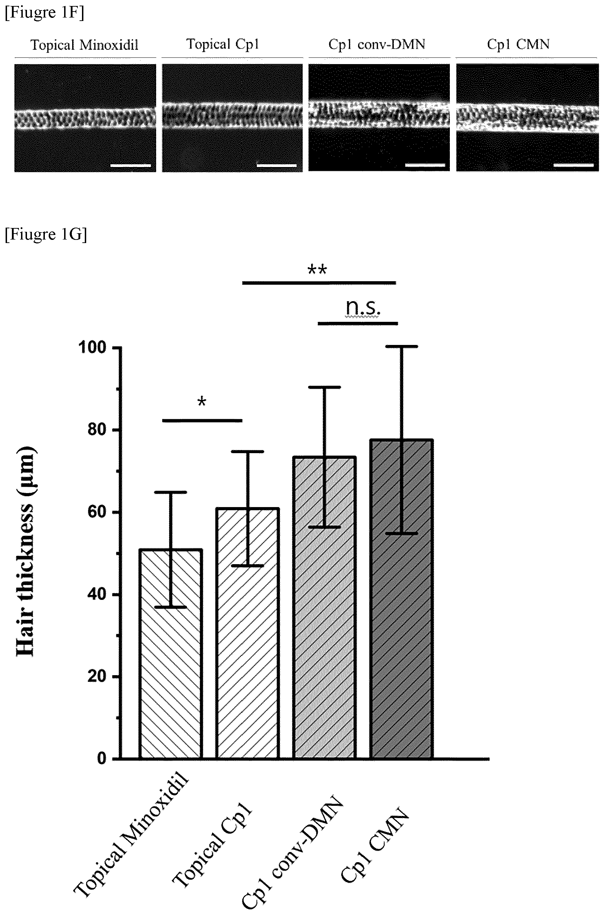

FIG. 1 is a result of comparing the hair growth effect and skin tissue development effect (FIG. 1A), the anagen phase conversion rate (FIG. 1B), the hair growth rate (FIG. 1C), the distribution rate by hair growth cycle (FIG. 1D), the skin thickness (FIG. 1E) and the change in the thickness of the hair (FIG. 1F & FIG. 1G), when Cp 1 is applied or injected using a microneedle.

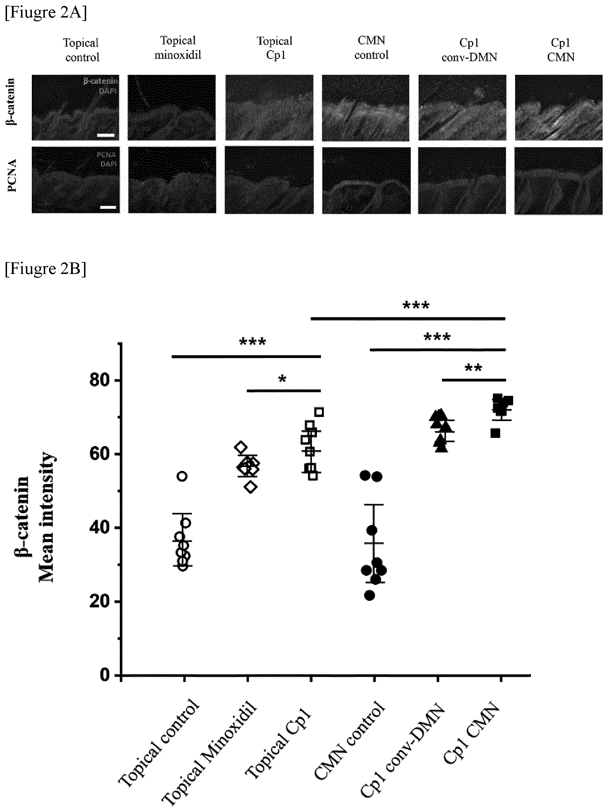

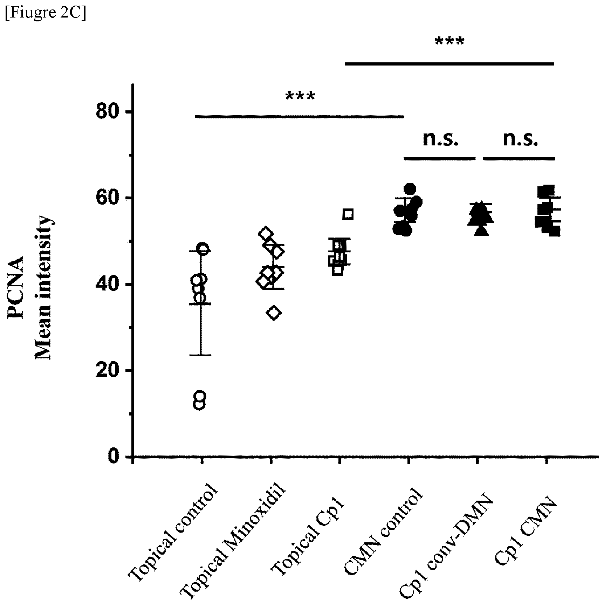

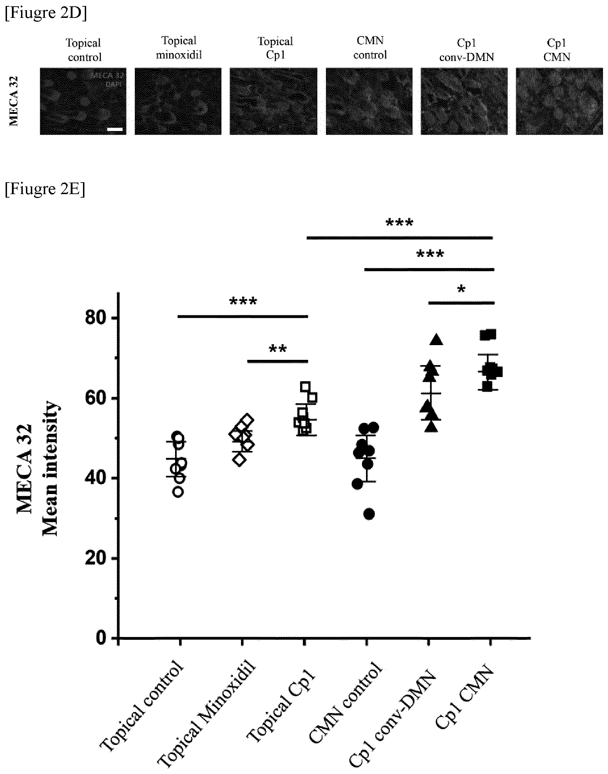

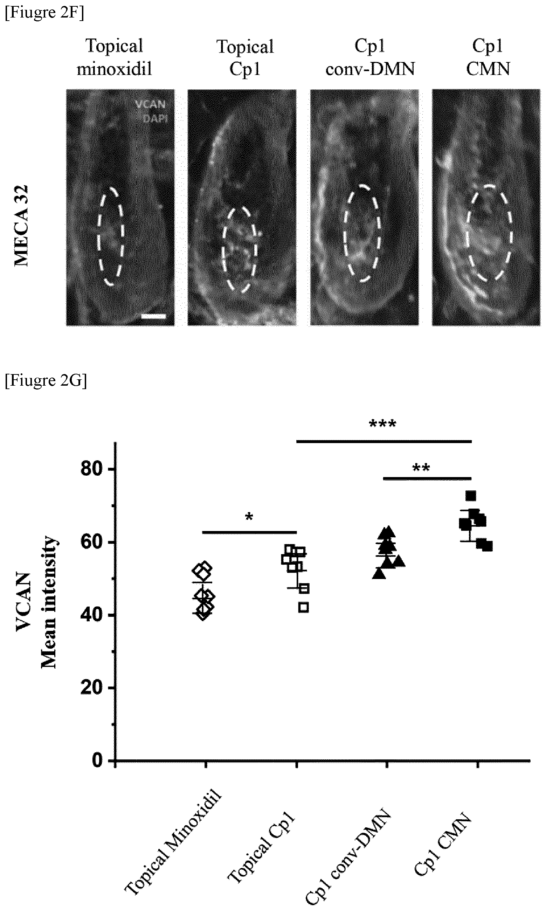

FIG. 2 is a result of analyzing the expression levels of skin tissue development markers β-catenin, PCNA, MECA32, and VCAN using fluorescence microscopy when Cp 1 is applied or injected using microneedles (FIG. 2A, 2D, 2F), and the corresponding quantitative analysis (Figures 2B, 2C, 2E, 2G).

### [Modes of the Invention]

Unless defined otherwise, all technical and scientific terms used herein have the same meanings as commonly understood by one of ordinary skill in the art to which the present invention pertains. In general, the nomenclature used herein and the experimental methods described below are those well-known and commonly used in the art.

In the present invention, it has been shown that the compounds respectively represented by Formula I have an effect on releasing NO and simultaneously inhibiting phosphodiesterase (PDES), exhibit an excellent therapeutic effect not only for male pattern baldness but also for female pattern baldness by this mechanism, and exhibit a remarkably excellent effect in hair loss and the like, compared to Minoxidil which is one of the most currently used drugs and available in the market.

In addition, it was demonstrated that when the compound represented by Formula **I** according to the present invention was administered before hair loss occurred, the effect of preventing hair loss could also be exhibited.

Thus, the present invention the present invention, in one aspect, relates to a transdermal pharmaceutical composition comprising a compound of Formula I or a pharmaceutically acceptable salt thereof. 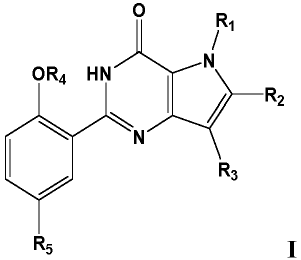 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, said compound of formula I is a compound, wherein R₁ is methyl or ethyl; R₂ is H; R₃ is ethyl or *n*-propyl; and R₄ is ethyl or *n*-propyl.

In the present invention, said compound of formula I is a compound, wherein R₁ is ethyl; R₂ is H; R₃ is *n*-propyl; and R₄ is *n*-propyl.

In the present invention, said compound of formula **I** is a compound selected from the group consisting of

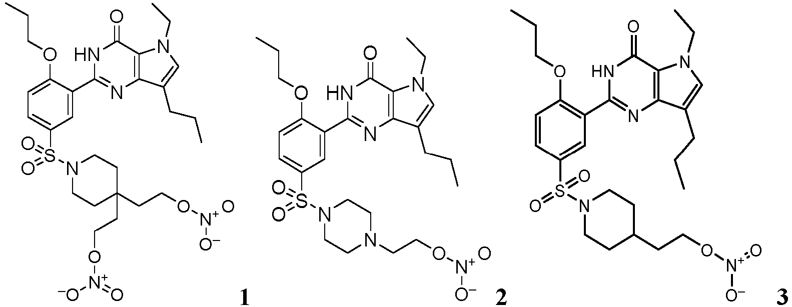

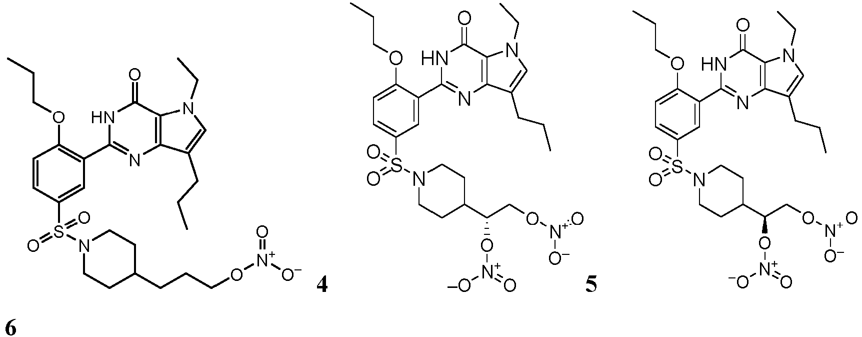

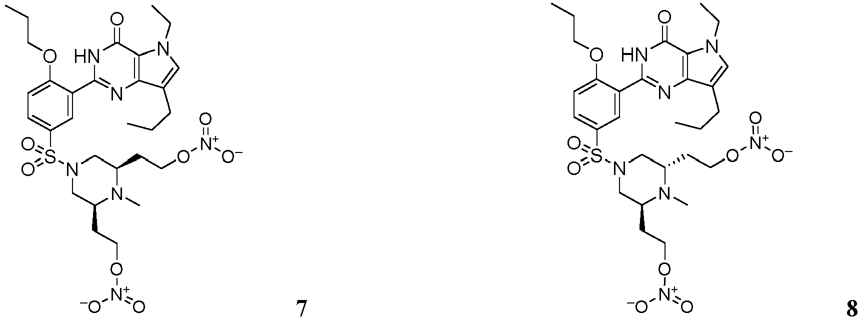

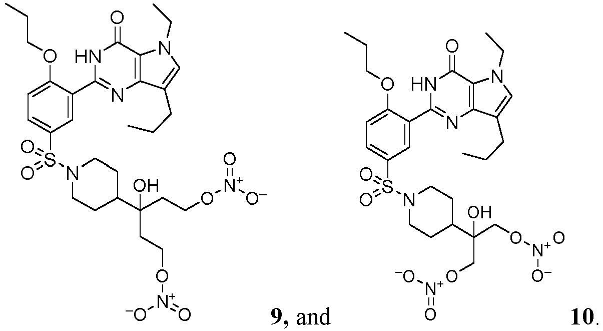

In the present invention, said compound of formula **I** is a compound selected from the group consisting of:
(1-((3-(5-ethyl-4-oxo-7-propyl-4,5-dihydro-3H-pyrrolo[3,2-d]pyrimidin-2-yl)-4-propoxyphenyl)sulfonyl)piperidine-4,4-diyl)bis(ethane-2,1-diyl) dinitrate **(1);**
2-(4-((3-(5-ethyl-4-oxo-7-propyl-4,5-dihydro-3H-pyrrolo[3,2-d]pyrimidin-2-yl)-4-propoxyphenyl)sulfonyl)piperazin-1-yl)ethyl nitrate **(2);**
2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethyl nitrate **(3);**
3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)propyl nitrate **(4);**
(R)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate **(5);**
(S)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate **(6);**
((2R,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate **(7);**
((2S,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate **(8);**
3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-3-hydroxypentane-1,5-diyl dinitrate **(9);** and
2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-2-hydroxypropane-1,3-diyl dinitrate **(10).**

In the present invention, the compound of Formula I or pharmaceutically acceptable salt thereof may include all compounds represented by Formula I, as well as their prodrugs, solvates, hydrates, metabolites, or pharmaceutically acceptable salts.

In a preferred aspect, said compound of formula **I** is Compound 1 (Cp 1) or Compound 2 (Cp 2):

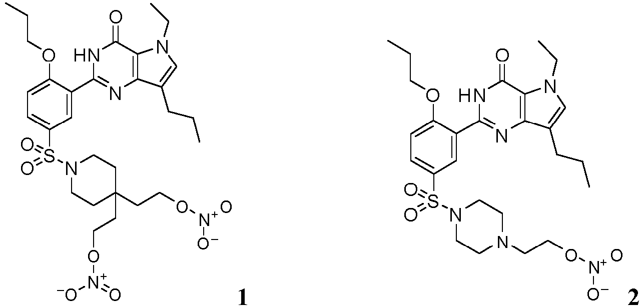

In a very preferred aspect, said compound of formula I is Compound 1.

For example, said compound Cp 1 represented by Formula I may undergo the following metabolic process *in vivo,* and in the present invention, it will be apparent to those skilled in the art that the effect of the invention may be exerted by administering a primary or secondary metabolite instead of the compound represented by Formula I .

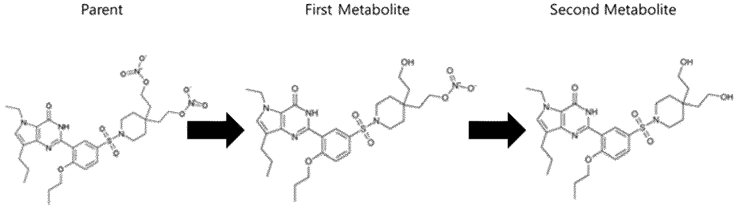

In addition, said compound 2(TOP-M111) represented by Formula I may undergo the following metabolic process *in vivo,* and in the present invention, it will be apparent to those skilled in the art that the effect of the invention may be exerted by administering the corresponding metabolite instead of the compound represented by Formula 1.

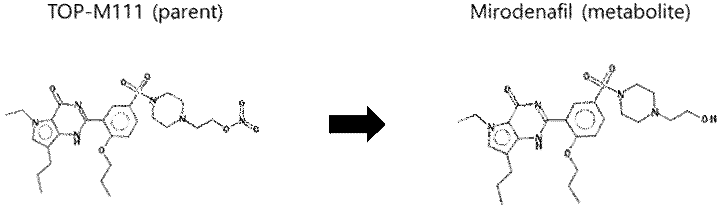

In the present invention, the pharmaceutical composition may be used to prevent, ameliorate, or treat one or more diseases or disorders selected from the group consisting of:
skin diseases, skin aging, steroid-induced skin atrophy;
erectile dysfunction; and
hair loss or hair growth,
but is not limited thereto.

In the present invention, the pharmaceutical composition may be characterized as being for topical administration, and preferably for transdermal administration, but is not limited thereto.

In the present invention, the pharmaceutical composition may be characterized in that the compound represented by Formula I, preferably said Compound 1 and compound 2 thereof or a pharmaceutically acceptable salt thereof is formulated in an amount of 0.00005 to 0.5% (w/w), but is not limited thereto. For example, the compound represented by Formula I or pharmaceutically acceptable salt thereof may be included in an amount of 0.00005 to 0.5%, 0.0001 to 0.04%, 0.0002 to 0.03%, or 0.0004 to 0.02%, but is not limited thereto. For example, the pharmaceutical composition may be formulated to comprise the compound represented by Formula I, preferably Compound 1 or Compound 2, or pharmaceutically acceptable salt thereof, in an amount of 0.0005% to 0.05% or 0.0008% to 0.004% (w/w).

In the present invention, the compound represented by Formula I, or a pharmaceutically acceptable salt thereof may be characterized in that it is formulated in an amount of 0.0005 to 0.02% (w/w) for administration to males, but is not limited thereto.

In the present invention, the compound represented by Formula I, preferably said Compound 1 or compound 2 or a pharmaceutically acceptable salt thereof may be characterized in that it is formulated in an amount of 0.00005 to 0.01% (w/w) for administration to females, but is not limited thereto.

In the present invention, the compound represented by Formula I, preferably said Compound 1 or compound 2 or a pharmaceutically acceptable salt thereof may prevent hair loss or promote hair growth by
(i) inducing anagen of hair follicles;
(ii) promoting melanogenesis;
(iii) increasing the number of hair follicles;
(iv) increasing skin thickness;
(v) promoting angiogenesis;
(vi) proliferating cells in outer root sheath; and/or
(vii) enhancing blood flow in the hair follicle, but is not limited thereto.

In the present invention, the hair loss may be characterized as androgenetic alopecia or chemotherapy-induced alopecia, or Alopecia areata but is not limited thereto.

In another aspect, the present invention provides a compound of formula I, or a pharmaceutically acceptable salt thereof for transdermal use. 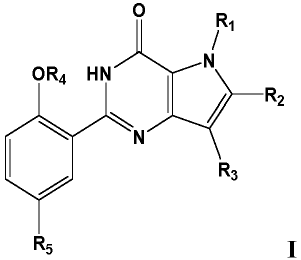 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, the transdermal use may be for preventing, ameliorating, or treating one or more diseases or disorders selected from the group consisting of:
skin diseases, skin aging, steroid-induced skin atrophy;
erectile dysfunction; and
hair loss or hair growth.

In another aspect, the present invention provides a use of a compound of formula I, or a pharmaceutically acceptable salt thereof in the manufacture of a transdermal medicine. 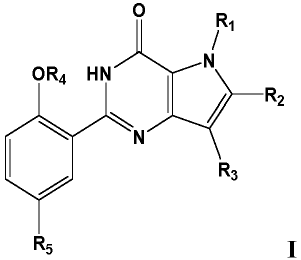 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, the medicine may be for use in preventing, ameliorating, or treating one or more diseases or disorders selected from the group consisting of:
skin diseases, skin aging, steroid-induced skin atrophy;
erectile dysfunction; and
hair loss or hair growth.

In another aspect, the present invention provides a transdermal administration method comprising administering a compound of Formula I or a pharmaceutically acceptable salt thereof to a subject in need thereof: 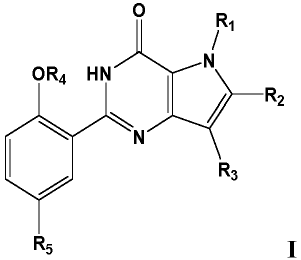 wherein
R₁ is C₁-C₃ alkyl, preferably methyl or ethyl, further preferably ethyl;
R₂ is H, CHO or CH=N-OH, preferably H, CHO or (*E*)-CH=N-OH, further preferably H;
R₃ is C₁-C₄alkyl, preferably ethyl or propyl, further preferably *n*-propyl;
R₄ is C₁-C₆alkyl, preferably ethyl or propyl, further preferably *n*-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

In the present invention, the transdermal administration method is for preventing, ameliorating, or treating one or more diseases or disorders selected from the group consisting of:
skin diseases, skin aging, steroid-induced skin atrophy;
erectile dysfunction; and
hair loss or hair growth.

The detailed descriptions of the use, the use for medicine manufacture, and transdermal administration method are referenced to the description of the composition, insofar as they are not mutually contradictory.

The compound according to the present invention may treat, impede or inhibit hair loss, and for example, it may be characterized in that it prevents hair loss or promotes hair growth by (i) inducing anagen of hair follicles, (ii) promoting melanogenesis, (iii) increasing the number of hair follicles, (iv) increasing skin thickness (including the development of skin tissues), (v) promoting angiogenesis, (vi) proliferating cells in outer root sheath, and/or (vii) enhancing blood flow in the hair follicle in a subject with hair loss. In the present invention, the hair loss may be due to androgenetic alopecia (AGA), and the hair loss may be due to male pattern baldness or female pattern baldness. In another embodiment, the hair loss may be the result of skin damage, and for example, the hair loss may be hair loss caused by drug or chemotherapy treatment.

In one aspect, the hair loss is scarring (or in another embodiment, scar) hair loss, which is hair loss due to scarring of the scalp area. The scarring hair loss typically occurs on the top of the scalp and is mainly seen in women. This condition is commonly seen in African-American women and is thought to be related to persistent tying or "corn-rowing" of the hair. In addition, the form of scarring hair loss may also appear in postmenopausal women associated with inflammation of the hair follicles and the resulting scarring.

In another aspect, the hair loss includes known baldness. In another aspect, the present invention is about alopecia areata as an autoimmune disorder that causes uneven hair loss or shows a large area of hair loss in the form of an "island" of hair remaining from extensive hair loss.

In another aspect, the hair loss includes hair loss due to compulsive hair pulling.

In another aspect, the hair loss includes triangular alopecia in which hair in the temporal lobe region begins to fall off in childhood. In the hair loss, hair loss may be completely progressed, or some fine and thin hair may remain.

In another aspect, the hair loss includes telogen alopecia, which is a common type of hair loss that occurs when a high percentage of hair has progressed to the "shedding" stage. The causes of telogen alopecia may be hormones, nutrients, drug-related or stress-related.

In another aspect, the hair loss includes loose-anagen syndrome, which is a symptom mainly seen in blondes, in which the scalp hair region is loose from the hair follicles and is easily pulled out by combing or pulling. This condition may appear in childhood.

In another aspect, the hair loss may be caused by fungal infection, and in one embodiment, tinea capitis (scalp ringworm) which is characterized by patches of dandruff and spreads from the scalp to cause hair breakage, redness, swelling and exudation may be treated.

In another aspect, the hair loss includes hair loss, which is associated with certain conditions such as cancer, thyroid disease, protein deficiency in the diet or low serum iron levels, or associated with certain environmental stimuli such as chemotherapy or other radiotherapy.

In another aspect, the present invention may treat any disease, disorder or symptom associated with baldness. In another aspect, the present invention may treat any disease, disorder or symptom associated with a degenerative skin disorder. In another aspect, the present invention may treat any disease, disorder or symptom associated with alopecia. Each disease, disorder or symptom is a separate embodiment of the present invention.

As used herein, the term "treatment" refers to therapeutic treatment or a prophylactic or preventive measure to prevent or ameliorate a targeted pathological condition or disorder. Thus, in one aspect, treatment may include directly acting on, curing, inhibiting, impeding, preventing, reducing the severity, delaying the onset, or alleviating the symptoms associated with the disease, disorder or condition, or a combination thereof. That is, in one aspect, the term "treatment" means delaying progression, promoting a decline, inducing a decline, enhancing a decline, promoting recovery, increasing efficacy or decreasing resistance to an alternative therapeutic agent or a combination thereof. In one aspect, the term "prevention" refers to delaying the onset of symptoms, preventing recurrences, reducing the frequency or number of recurrences, increasing the latency between symptom occurrences or a combination thereof. In one embodiment, the term "inhibition" or "impediment" refers to alleviating the severity of symptoms, alleviating the severity of the current episode, reducing the number of symptoms, reducing the incidence of symptoms, reducing the latency of symptoms, alleviating symptoms, reducing secondary symptoms, reducing secondary infections or a combination thereof.

In one aspect, the symptom is primary, and in another aspect, the symptom is secondary. In one aspect, the term "primary" refers to a symptom that is a direct result of alopecia, and in one aspect, the term "secondary" refers to a symptom that results from or is a result of a primary cause. In one aspect, the compositions and strains for use in the present invention co-treat alopecia, and in one aspect, they treat primary or secondary symptoms or secondary complications associated with seborrheic dermatitis.

In another aspect, the term "symptom" may be any symptom of hair loss, including hair loss, baldness, temporary hair loss, patchy hair loss, degenerative skin disorders or a combination thereof.

Methods for determining the presence and severity of hair loss as described herein are well known in the art. Each method is a separate embodiment of the present invention.

In one aspect, the method of the present invention is for treating a subject with hair loss. In another aspect, the hair loss is hair loss on the scalp of the subject. In another aspect, the hair loss is hair loss of the eyebrow of the subject. In another embodiment, the hair loss is hair loss in the subject's scarred skin tissue, which may be the subject's scalp, eyebrows, arms or legs in one embodiment. In another embodiment, any other area or region of the skin where there is body hair may be treated by the method of the present invention. Each possibility is a separate embodiment of the present invention.

In some aspects of the present invention, the pharmaceutical composition comprising the compound represented by Formula I, or a pharmaceutically acceptable salt thereof as an active ingredient may use any one formulation selected from the group consisting of injection, granules, gels, suspensions, emulsions, drops or liquids according to conventional methods, and more preferably, it may be used as an external preparation or injection.

In another aspect of the present invention, the pharmaceutical composition comprising the compound represented by Formula I thereof or a pharmaceutically acceptable salt thereof as an active ingredient may include one or more additives selected from the group consisting of carriers, excipients, disintegrants, sweeteners, coating agents, swelling agents, lubricants, glydents, flavoring agents, antioxidants, buffers, bacteriostats, diluents, dispersants, surfactants, binders and lubricants, which are suitable and conventionally used in the preparation of pharmaceutical compositions.

Specifically, as the carriers, excipients and diluents, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil may be used. In addition, examples of solid preparations for oral administration may include tablets, pills, powders, granules, capsules and the like, and the solid preparations may be prepared by mixing at least one excipient, for example, starch, calcium carbonate, sucrose or lactose, gelatin and the like in the composition. In addition to simple excipients, lubricants such as magnesium stearate and talc may also be used.

In some aspects of the present invention, the pharmaceutical composition may be administered to a subject in via transdermal route by conventional means and preferably may be administered to the subject via hypodermic injection.

A preferred dosage of the compound represented by Formula I, or a pharmaceutically acceptable salt thereof, or a precursor of the compound may vary depending on the condition and weight of the subject, the type and degree of disease, drug form, administration route and duration, and it may be appropriately selected by those skilled in the art.

In one aspect of the present invention, the daily dose by application of the pharmaceutical composition may be administered at 0.1 to 1 mg/kg, more preferably, 1 to 100 µg/kg, and most preferably, 5 to 50 µg/kg, but is not limited thereto. In this case, about 0.1 to 10% of the drug may be administered in the body.

In one aspect, the concentration of the active ingredient may be 1 to 100 µM based on the total volume of the composition, and for example, 10 µM or more, 15 µM or more, 20 µM or more, 25 µM or more, 30 µM or more, 35 µM or more, or 40 µM or more, or it may be 90 µM or less, 80 µM or less, 70 µM or less or 60 µM or less. In one embodiment of the present specification, preferably, the concentration of the active ingredient may be 50 µM.

As another preferred aspect, the composition may be formulated as a cosmetic composition or a topical skin preparation, in addition to being a pharmaceutical composition.

In the present invention, the cosmetic composition or the topical skin preparation may be characterized in that it exhibits an effect of preventing or ameliorating hair loss, promoting hair growth, preventing or ameliorating skin disease, and preventing or ameliorating erectile dysfunction.

In the present invention, the composition may be characterized in that it is formulated in one or more forms selected from the group consisting of a softening lotion, an astringent lotion, a nourishing lotion, nourishing cream, massage cream, an essence, cleansing cream, a cleansing foam, cleansing water, a hair tonic, a hair conditioner, a hair essence, a hair lotion, a hair nutrition lotion, a hair shampoo, a hair conditioner, a hair treatment, hair cream, hair nourishing cream, hair moisture cream, hair massage cream, hair wax, hair aerosol, a hair pack, a hair nutrition pack, a hair soap, a hair cleansing foam, hair oil, a hair dryer, hair preservative, a hair dye, a hair wave agent, a hair bleach, hair gel, a hair glaze, a hair dressing, a hair lacquer, a hair moisturizer, a hair mousse and a hair spray, but is not limited thereto.

The appearance of the cosmetic composition according to an exemplary embodiment of the present invention contains a cosmetically or dermatologically acceptable medium or base. It is in any dosage form suitable for topical application, for example, it may be provided in the form of solutions, gels, solids, kneaded dry products, emulsions obtained by dispersing the oily phase in an aqueous phase, suspensions, microemulsions, microcapsules, microgranules or ionic types (liposomes) and a non-ionic vesicular dispersant, or it may be provided in the form of cream, skin, lotions, powders, ointments, sprays or concealer sticks. These compositions may be prepared according to conventional methods in the art. The composition according to the present invention may also be used in the form of a foam or in the form of an aerosol composition further containing a compressed propellant.

In some aspect of the present invention, the cosmetic composition is not particularly limited in its formulation, and may be formulated into cosmetic products, for example, a softening lotion, astringent lotion, nutrient lotion, nourishing cream, massage cream, essence, cleansing cream, cleansing foam, cleansing water, hair tonic, hair conditioner, hair essence, hair lotion, hair nourishing lotion, hair shampoo, hair conditioner, hair treatment, hair cream, hair nourishing cream, hair moisture cream, hair massage cream, hair wax, hair aerosol, hair pack, hair nutritional pack, hair soap, hair cleansing foam, hair oil, hair dryer, hair preservative, hair dye, wave agent for hair, hair bleach, hair gel, hair glaze, hair dresser, hair lacquer, hair moisturizer, hair mousse, hair spray or the like.

When the formulation of the present invention is a paste, cream or gel, animal fibers, vegetable fibers, wax, paraffin, starch, tragacanth, cellulose derivatives, polyethylene glycol, silicone, bentonite, silica, talc, zinc oxide or the like may be used as a carrier component.

When the formulation of the present invention is a powder or a spray, lactose, talc, silica, aluminum hydroxide, calcium silicate or polyamide powder may be used as a carrier component, and in particular, in the case of a spray, it may additionally include propellants such as chlorofluorohydrocarbons, propane/butane or dimethyl ether.

When the formulation of the present invention is a solution or an emulsion, a solvent, solvating agent or emulsifying agent may be used as a carrier component, and for example, there are water, ethanol, isopropanol, ethyl carbonate, ethyl acetate, benzyl alcohol, benzyl benzoate, propylene glycol, 1,3-butylglycol oil, glycerol fatty esters, or fatty acid esters of polyethylene glycol or sorbitan.

When the formulation of the present invention is a suspension, water, a liquid diluent such as ethanol or propylene glycol, a suspending agent such as ethoxylated isostearyl alcohol, polyoxyethylene sorbitol esters and polyoxyethylene sorbitan esters, microcrystalline cellulose, aluminum metahydroxide, bentonite, agar or tragacanth may be used as a carrier component.

When the formulation of the present invention is a surfactant-containing cleansing agent, aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkylamidobetaine, aliphatic alcohol, fatty acid glyceride, fatty acid diethanolamide, vegetable oil, a lanolin derivative, ethoxylated glycerol fatty acid ester or the like may be used as a carrier component.

In some aspect of the present invention, the cosmetic composition may further include functional additives and components included in general cosmetic compositions in addition to the active ingredient. As the functional additives, a component selected from the group consisting of water-soluble vitamins, oil-soluble vitamins, high-molecular peptides, high-molecular polysaccharides, sphingolipids and seaweed extract may be included, and other cosmetic compositions for which hair loss prevention or hair growth effects are confirmed may be further included.

In the cosmetic composition of the present invention, a component contained in a general cosmetic composition may be further blended with the functional additives as needed. Examples of other included blending ingredients include oils and fats, moisturizers, emollients, surfactants, organic and inorganic pigments, organic powders, UV absorbers, preservatives, fungicides, antioxidants, plant extracts, pH adjusters, alcohols, pigments, fragrances, blood circulation accelerators, cooling agents, limiting agents, purified water and the like.

The composition according to one aspect of the present invention may be an external preparation for skin. The external preparation for skin is a generic term that may include anything applied outside the skin, and various formulations of cosmetic and pharmaceutical products may be included therein.

In another aspect, the present invention relates to a kit for the preparation of a formulation for topical skin application, wherein the pharmaceutical or cosmetic composition is included. The kit is designed for preparing a transdermal formulation for application to the skin, preferably human skin, and may comprise an adhesive or non-adhesive patch or device.

In one aspect, the kit comprises:
(i) a first kit component comprising the pharmaceutical composition; and
(ii) a second kit component comprising a patch or device, either adhesive or non-adhesive, suitable for applying the pharmaceutical composition to a skin.

In another aspect, the present invention relates to a solid composition for formulating the composition into a liquid composition, and the solid composition may be characterized in that it is soluble in an aqueous liquid solvent, and the liquid composition comprises 0.00005 to 0.5% (w/w) of a compound represented by Formula I, its derivatives or a pharmaceutically acceptable salt thereof.

Meanwhile, the pharmaceutical composition or cosmetic composition according to the present invention may be administered to the skin, scalp, or other areas where treatment or hair growth is desired, in the form of microneedles.

Accordingly, in another aspect, the present invention relates to a microneedle structure on which the pharmaceutical composition or cosmetic composition is mounted.

In the present invention, the microneedle structure may be a microneedle patch or a microneedle applicator, but is not limited thereto. In the present invention, the microneedle may include a biocompatible polymer selected from the group consisting of hyaluronic acid, collagen, polyvinyl pyrrolidone, polylactic acid, polylactic glycolic acid, polylactide, polyglycolic acid, polyethylene glycol, polycaprolactone, polyamino acid, polypropylene fumarate, polygama-glutamic acid, polyvinyl alcohol, polyethylene imine, albumin, dextran, fibrin, elastin, gelatin, alginic acid, polyacrylic acid, carboxymethyl cellulose, and mixtures or copolymers thereof, but is not limited to.

As used herein, the term "biocompatible polymer" composition refers to a material that is not particularly toxic to the human body, is chemically inert and has no immunogenicity, and preferably a polymer composition that can be decomposed by body fluids or microorganisms *in vivo* may be used.

In the present invention, the microneedle structure may be characterized that examples thereof include solid microneedles, coated microneedles, dissolving microneedles, hollow microneedles or candlelit-shaped microneedles, and preferably, it may be a candlelit-shaped microneedle, but is not limited thereto.

In the present invention, the microneedle may further include a viscous composition.

As used herein, the term "viscous composition" refers to a composition having the ability to form a microstructure by extension in the negative pressure forming direction when applying a negative pressure used in the present invention. The viscous material used in the present invention includes an acrylic polymer, an amide polymer, an acetyl polymer, a vinyl polymer, an epoxy polymer, a silicone polymer, a sulfone resin, a polycarbonate polymer or a copolymer thereof, but is not limited to, and any viscous material commonly used in the art may be used.

Preferably, the viscous material used in the present invention has viscosity when fluidized. This viscosity may be variously changed according to the type, concentration and temperature of the viscous material, the organic solvent and the like, and it may be appropriately adjusted for the purpose of the present invention. More preferably, the viscous material of the present invention exhibits a viscosity of 200,000 cSt (centistoke) or less when fluidized.

The viscosity of such a viscous composition may be variously changed according to the type, concentration and temperature of the material or the addition of a thickener included in the composition, and it may be appropriately adjusted for the purpose of the present invention. The viscosity of the viscous composition may be adjusted by the intrinsic viscosity of the viscous material, and may also be adjusted by using an additional thickening agent in the viscous composition.

For example, thickeners commonly used in the art, for example, thickeners such as hyaluronic acid and a salt thereof, polyvinylpyrrolidone, a cellulose polymer, dextran, gelatin, glycerin, polyethylene glycol, polysorbate, propylene glycol, povidone, carbomer, gum ghatti, guar gum, glucomannan, glucosamine, dammer resin, rennet casein, locust bean gum, microfibrillated cellulose, psyllium seed gum, xanthan gum, arabino galactan, gum arabic, alginic acid, gelatin, gellan gum, carrageenan, karaya gum, curdlan, chitosan, chitin, tara gum, tamarind gum, tragacanth gum, furcelleran, pectin or pullulan may be added to the main component of a solid microstructure, for example, the composition including a biocompatible material to adjust the viscosity to be suitable for the present invention.

In the present invention, the composition may be injected once within 2 to 30 days, for example, once in 2 days, once in 3 days, once in 4 days, once in 5 days, once in 6 days, once in 7 days, once in 8 days, once in 9 days, once in 10 days, once in 11 days, once in 12 days, once in 13 days, once in 14 days, once in 15 days or once every 30 days, but is not limited thereto.

In the present invention, Korean Patent Laid-Open Nos. 10-2016-0024354, 10-2020-0024515 and 10-2020-0085224, and Korean Registered Patent No. 10-2175312 relating to microneedles are incorporated herein by reference, and those skilled in the art to which the present invention pertains may easily administer the pharmaceutical composition or cosmetic composition according to the present invention to a region where hair growth is desired using the microneedles described in the above-published patents and other known microneedles. In a preferred aspect, the microneedle may be a biodegradable microneedle (DMN; dissolving microneedle), but is not limited thereto.

In the present invention, the microneedles may be solid microneedles, coated microneedles, dissolving microneedles or hollow microneedles.

Solid microneedles may be generally used for the use of skin pre-treatment applications, but is not limited thereto. When a micrometer (µm)-sized hole is formed on the skin surface after inoculation with a solid microneedle, the drug is applied over the hole or a patch containing the drug is applied such that the drug may be absorbed into the body through the hole and delivered into the skin.

Coated microneedles are a type of microneedles in which, after the drug is mounted on the microneedles by coating the drug using a water-soluble agent such as a viscosity increasing agent, a surface treatment agent and the like on the surface of the microneedles, the coated drug is dissolved and delivered when inoculated into the skin. As coated microneedles, sharp microneedles made of metal materials are generally used that enable effective inoculation, but these are not suitable for drugs that require a large amount of mounting because they are small, and these are suitable for loading vaccines that are effective with only a small amount.

Dissolving microneedles are in the form of mounting drugs using biodegradable polymers or saccharides inside the needle, and have an advantage that these are completely dissolved into the inoculation site during transdermal inoculation and do not require a separate treatment after inoculation with the microneedles.

Hollow microneedles are a general syringe type of hollow microneedles, and are easy to deliver liquid formulations or a large amount of drugs or vaccines.

In the present invention, in-plane type microneedles (Lin Liwei, et al., "Silicon-processed Microneedles", Journal of Microelectromechanical Systems: a joint IEEE and ASME publication on microstructures, microactuators, microsensors, and microsystems 8(1): 78-84(1999)) may be used.

In another aspect for injecting the compound or pharmaceutical composition, biodegradable polymer microneedles *(e.g.,* Jung-Hwan Park, et al., "Biodegradable polymer microneedles: Fabrication, mechanics and transdermal drug delivery", Journal of Controlled Release 104 (1):51-66 (2005)) may be used.

In another aspect for injecting the compound or pharmaceutical composition, absorbable microneedles (*e.g*., Japanese Patent Application Laid-Open No. 2005154321; and Takaya Miyano, et al., "Sugar Micro Needles as Transdermic Drug Delivery System", Biomedical Microdevices, 7(3): 185-188(2005)) may be used.

In another aspect for injecting the compound or pharmaceutical composition, biodegradable microneedles (*e.g.,* Sean P Sullivan, et al., "Minimally Invasive Protein Delivery with Rapidly Dissolving Polymer Microneedles", Advanced Materials 20(5):933- 938 (2008)) may be used.

In another aspect for injecting the compound or pharmaceutical composition, hollow microneedles (*e.g*., Korean Patent Laid-Open No. 10-2016-0024354) may be used.

In another aspect for injecting the compound or pharmaceutical composition, biodegradable polymer microneedles suitable for quantitative delivery of a drug (*e.g.,* Korean Patent Laid-Open No. 10-2020-0024515) may be used.

In addition, as a form for injecting the compound or pharmaceutical composition, microneedles manufactured using a centrifugal force *(e.g.,* PCT Patent No. PCT/KR2014/003964) may be used.

Meanwhile, as the most preferred form for injecting the compound or pharmaceutical composition, candlelit-shaped microneedles (*e.g.,* Korean Patent Laid-Open No. 10-2175312) may be used.

A preferred candlelit-shaped microneedle according to the present invention includes a tip portion including a drug; a base portion formed on one side of the tip portion; and a separation portion formed between a substrate and a base portion and separating the substrate and the base portion after the tip portion is inserted into the skin, which physically separates by an external force or chemically separates by a chemical substance, wherein the base portion provides a supporting force such that the tip portion can be completely inserted into the skin, and the tip portion may be a microstructure having a candle-shaped cross section.

In the present invention, the drug may be a compound, a pharmaceutical composition or a cosmetic composition according to the present invention.

In the present invention, the separation portion may be a microstructure including at least one selected from biodegradable polymers and saccharides.

In the present invention, the time required for the separation portion to be dissolved by the chemical substance may be less than 5 minutes.

In the present invention, 70% or more of the separation portion per unit volume may be dissolved by the chemical substance within 3 minutes.

In the present invention, the chemical substance may be introduced between the substrate and the skin or may be applied on the skin before the tip portion is inserted into the skin.

In the present invention, the chemical substance includes at least one selected from water, an aqueous solution and an injectable ester, and the aqueous solution includes at least one selected from an anhydrous or hydrous lower alcohol having 1 to 4 carbon atoms, acetone, ethyl acetate, chloroform, 1,3-butylene glycol, nucleic acid, diethyl ether and butyl acetate, and the injectable ester includes at least one selected from a water-insoluble agent, propylene glycol, polyethylene glycol, an oily component, and ethyl oleate, and the oil that may be used as an oily component may include any one or more selected from vegetable oil, mineral oil, silicone oil and synthetic oil.

In the present invention, the cross-section of the central portion of the separation portion may have a smaller area than the cross-sections of both sides of the separation portion.

In the present invention, the separation portion may be broken by a force smaller than the bonding force with any one of the base portion and the substrate.

In the present invention, the separation portion may be broken by a force smaller than the force that the tip portion is separated from the skin.

In the present invention, the separation portion may have a strength of 0.01 to 1N.

In the present invention, the external force may be applied in a horizontal direction or a vertical direction with respect to the separation portion.

In the present invention, the separation portion or the base portion may include a biodegradable material.

In the present invention, the substrate may be formed with a protrusion having a predetermined thickness in a portion corresponding to the separation portion formed on one surface thereof.

In the present invention, the protrusion may be formed in a horizontal straight line, an inclined oblique line, or a convex or concave curve in the cross section of the surface on which the separation portion is formed

As used herein, the term "effective amount" refers to an amount necessary or sufficient to implement a desired biological effect. Preferably, the term "effective amount" refers to an amount of the compound of Formula (1) of the present invention, which is capable of (i) treating or preventing a particular disease, medical condition, or disorder, (ii) weakening, alleviating or eliminating one or more symptoms of the particular disease, medical condition, or disorder, or (iii) preventing or delaying the onset of one or more symptoms of the particular disease, medical condition, or disorder described in the present specification. An effective amount of the compound of the present invention of Formula (1) or the pharmaceutical composition will be an amount that achieves this selected result, and such amount may be determined as a routine matter by one of ordinary skill in the art. In addition, preferably, as used herein, the term "effective content" typically and preferably refers to an amount which is necessary or sufficient to increase releasing NO and the inhibition of PDE5. The effective content may vary depending on the particular composition administered and the size of the subject. One of ordinary skill in the art may empirically determine the effective amount of the particular composition of the present invention without the need for undue experimentation.

As used herein, the term "mammal" includes humans, mice, rats, guinea pigs, monkeys, dogs, cats, horses, cattle, pigs and sheep, but is not limited thereto. As used herein, the term "mammal" preferably refers to a human.

The term "pharmaceutically acceptable" refers to properties that do not impair the biological activity and properties of a compound.

As used herein, the term "prevention" refers to any action of preventing the onset of a disease or inhibiting its progression by administration of a composition including the compound.

As used herein, the term "amelioration" refers to any action that at least reduces a parameter, for example, the severity of a symptom, related to the treated state of a disease by administration of a composition including the compound.

In addition, as used herein, the term "treatment" refers to any action in which symptoms of a disease are ameliorated or cured by administration of a composition including the compound.

As used herein, the term "administration" refers to the act of introducing the pharmaceutical composition of the present invention to a subject by any suitable method, and the administration route may be administered through various oral or parenteral routes as long as it can reach the target tissue.

As used herein, the term "topical administration" is used broadly to include administration to a body surface that is generally open to the surroundings. It includes the skin as well as the nasal and oral passages and the genitals. Thus, topical administration includes application to the skin, application to the nasal cavity, application to the oral cavity (including the upper throat), and administration to the genital organs. Topical formulations are available in a variety of forms and include creams, ointments, solutions, lotions, suspensions, pastes, emulsions, foams and the like. Water-miscible creams are generally applied to moist and oozing lesions, and ointments are generally chosen for dry, lichenified or scaly lesions or wounds that require more occlusive effect. Lotions are generally useful when minimal application to large or hair areas is required or for treating exudative lesions. The term "local administration" is used herein to refer to topical administration and eye drop administration.

As used herein the term "% (w/w)" refers to (mass of the component / total mass of the composition) × 100.

As used herein, "transdermal" administration in the present invention refers to a method of delivering a drug through the skin, "hypodermic" injection refers to administering a drug by injecting it into the subcutaneous tissue. To prevent the side effects of systemic hypotension caused by PDE5 inhibitors, it is preferable to administer the composition locally to the site of action. Transdermal administration is even more preferable, and hypodermic injection is the most preferred method. As the most preferred embodiment of local application, transdermal administration, or hypodermic injection, a microneedle patch in the form of a candle containing Compound 1 can be used.

As used herein, the term "kit" means that the components comprised in said kit are provided physically separable and distinguishable from one another as different components but are provided or sold together for the purpose of being administered, or used, together.

The compound or transdermal composition according to the present invention may be administered by a suitable route, for example, topical, or transdermal, Other routes are known in the art and may also be applied, for example, by surgical entry. Thus, equipment for administration, such as conventional needles and syringes, microneedles, patches *(e.g.,* WO 98/20734), needle-free injection systems (*e.g.,* WO 1999027961 A1), spraying equipment and the like may be used depending on the dosage form and the route of administration. In the equipment, the compound or pharmaceutical composition of the present invention may be pre-filled or coated.

### Example

Hereinafter, the present invention will be described in more detail through examples. These examples are only for illustrating the present invention, and it will be apparent to those of ordinary skill in the art that the scope of the present invention is not to be construed as being limited by these examples.

### Synthesis of Compounds of represented by Formula I

Compounds of Formula I as well as their syntheses are disclosed in WO/2018/215433, the disclosure of which is herewith incorporated by reference in its entirety. In particular Examples **1n** and **1i** of WO/2018/215433 contain a detailed description of the preparation of preferred compounds of formula I.

### Example 1

### Preparation of Cp 1-loaded candlelit microneedle (CMN)

Candlelit microneedle (CMN) was fabricated in two main steps: fabrication of base layer and head layer. The base layer, which was composed of biodegradable polymer, was fabricated using contact-and-dry method. After dispensing 20% (w/v) of pharmaceutical grade hyaluronic acid aqueous solution (PHA, 30 kDa; Youthcarefarm, Suwon, Republic of Korea) on a base plate, the droplets were placed in contact with a cover plate. The droplet was formed into a wine glass shape by natural drying with a constant 300 µm distance between the two plates. Next, 60% (w/v) PHA droplets were dispensed on 4% (w/v) carboxymethylcellulose coated film (CMC, 90 kDa; Sigma-Aldrich, St. Louis, MO, USA). Further, the CMN was fabricated by centrifugal lithography at 202 g force for 1 min. The PHA droplets loaded 0.027% (w/v) of Cp 1 and final Cp 1 CMN fabricated by this solution contained 2 µg drug per 5 × 5 array.^{[20]} *Applicator:* The microneedle applicator that supports penetration of CMN was designed for application on the scalp, which is a hairy part of the skin. Micropillars in the device were aligned in 5 × 5 CMN arrays, which provided an insertion force for penetrating the hairy skin.

### Example 2

### Preparation of Cp 1-loaded conventional-shaped dissolving microneedl e (conv-DMN)

2-1. Conv-DMN having conical shape loaded with Cp 1 was fabricated using the same polymers, drug loading amount of Cp 1 (2 µg per day)., and height (700 µm) as that of Ex. 1.

2-2. Conv-DMN having conical shape loaded with Cp 1 was fabricated using the same polymers, drug loading amount of Cp 1 (4 µg per day), and height (700 µm) as that of Ex. 1.

*High performance liquid chromatography (HPLC) conditions for CP 1:* A reverse-phase HPLC (Waters 600S; Waters, Milford, MA, USA) with a C18 column (150 mm × 4.6 i.d., Cosmosil 5C18-AR-II; Nacalai Tesque Inc., Kyoto, Japan) for quantitative analysis of Cp 1 loading amount in CMN was used. To set a calibration curve, a preparation of a stock solution of Cp 1 and sequentially diluted the solution from 1.5 mg/mL to 0 (R² ≥ 0.99). A mobile phase was prepared with 50% (v/v) acetonitrile and 50% (v/v) distilled water and the flow rate was set at 1 mL/min. The detection wavelength of Cp 1 was 254 nm. The retention time was determined to be 6.625 min.

### Example 3 . Enhancement effect in injection of Cp 1 using microneedles

### 3-1. Experimental method

7-week-old C57BL/6 mice (Orient Bio, Korea) with hair follicles in the telogen phase were purchased and the experiment was performed. The experiment was conducted using 4 mice each for 6 experimental groups of a vehicle-applied control group (topical control), a Minoxidil-applied positive control group (topical Minoxidil), an Cp 1-applied group (topical Cp 1), a candlelit-shaped microneedle control group (CMN control), an Cp 1 microneedle administration group (Cp 1 conv-DMN) and an Cp 1 candlelit-shaped microneedle administration group (Cp 1 CMN).

**[Table 1]**

| Group | Treatment Group | | Number of Animals | |
|---|---|---|---|---|
| | | | Male | Female |
| 1 | Minoxidil-applied positive control group | 2%(w/v) | - | 4 |
| 2 | Cp 1 | 0.002%(w/v) (medium dose) | | 4 |
| | | CP 1 -applied group | - | |
| 3 | | CP 1 microneedle administration group (2 µm dose of drug mounted when applied once) | - | 4 |
| 4 | | Candlelit-shaped microneedle control group (2 µm dose of drug mounted when applied once) | - | 4 |
| 5 | Vehicle | PEG400 : EtOH = 7:3 | | 4 |
| 6 | Candlelit-shaped microneedle control group | Candlelit-shaped microneedle mounted only with vehicle | | 4 |

### Preparation of topical minoxidil and topical Cp-1 solutions:

In the topical formulation, polyethylene glycol 400 (PEG 400; Sigma-Aldrich, St. Louis, MO, USA) and ethanol (99.5°; Sigma-Aldrich) were mixed in 50 µL solution with a ratio of 7:3 (v/v) containing 2% (w/v) of minoxidil (Sigma-Aldrich) and 0.002% (w/v) of Cp-1 (2 µg per day). The topical formulation groups' 50 µL solutions were administered twice a day.

The experiment was started in a pink skin condition by removing the hair on the back of the mice, and the drug application was carried out twice a day for 21 days, and when injected using a microneedle, it was injected once every 2 days and was not injected on last Day 21.

### 3-2. Hair growth effect and skin thickness

On Day 21 of the administration, the back of each mouse was visually observed, and then the mice were sacrificed and the back tissue was isolated, prepared as a frozen tissue section and stained with H&E. As a result, it was confirmed that the Minoxidil-applied positive control group (topical Minoxidil), the Cp 1 application group (topical Cp 1), the Cp 1 microneedle administration group (Cp 1 conv-DMN) and the Cp 1 candlelit-shaped microneedle administration group (Cp 1 CMN) showed increased skin thicknesses along with the hair growth effect, and the dermal tissue in which the hair follicles were located developed, and the hair follicles developed. In this case, Cp 1 treatment was more effective than Minoxidil treatment, and it was found that the hair growth effect and the skin tissue development were more excellent when Cp 1 was injected using a microneedle compared to topical application. Meanwhile, when a candlelit-shaped microneedle was used, the best effect was exhibited (FIG. 1A).

### 3-3. Comparison of conversion rates of anagen phase

On Day 21 of the administration, the area in which the color of the back of the mice was changed to black, that is, the area converted to the anagen phase among the shaved areas was analyzed with the naked eye and the Image J software (National Institutes of Health, Bethesda, MD, USA), which is an image analysis program.

As a result of quantifying the skin area converted to black with respect to the total hair-removed skin area as a percentage, the area where the color of the back of the mouse was changed to black was 65.3 ± 22.3% in the Minoxidil-applied positive control group (topical Minoxidil), whereas 92.1 ± 3.0% of the shaved area was changed to black in the Cp 1application group (topical Cp 1), and it was confirmed that Cp 1had a better effect of converting to the anagen phase when applied compared to Minoxidil. Meanwhile, when Cp 1was administered with a microneedle, the effect of converting to the anagen phase was exhibited to a degree similar to that of topically applying Cp 1(FIG. 1B).

### 3-4. Comparison of hair growth rates

On Day 21 of the administration, the ratio of actual hair growth on the back of the mice was analyzed with the naked eye and the Image J software (National Institutes of Health, Bethesda, MD, USA), which is an image analysis program.

As a result of quantifying the skin area with actual hair growth as a percentage of the total hair removal skin area, hair growth occurred at 38.4 ± 17.1% in the Minoxidil-applied positive control group (topical Minoxidil) and at 61.0 ± 14.0% in the Cp 1application group (topical Cp 1), and it was found that Cp 1 had a more improved effect on hair growth compared to Minoxidil. Also, in the Cp 1candlelit-shaped microneedle administration group (Cp 1 CMN), the actual hair growth was observed at 92.9 ± 11.2%, and it was confirmed that most of the actual hair grew at a fast rate in the area converted to the anagen phase (FIG. 1C).

### 3-5. Comparison of distribution rates by hair growth cycles

On Day 21 of the administration, the distribution rate by hair growth cycle in mice was compared. To this end, the hair cycle was analyzed in the form of each stained hair follicle based on the H&E-stained image in Example 1-2. Five tissue sections were isolated from each of the four mice for each experimental group and analyzed, and the ratio of the telogen phase to the anagen phase in the form of hair follicles was confirmed with reference to the contents described in Lahiji, Shayan Fakhraei, et al. "Transcutaneous implantation of valproic acid-encapsulated dissolving microneedles induces hair regrowth." Biomaterials 167 (2018): 69-79.

As a result, 52.5 ± 1.3% of the Minoxidil-applied positive control group (topical Minoxidil) was converted to the anagen phase, whereas 59.3 ± 3.0% of the Cp 1application group (topical Cp 1) was converted to the anagen phase, and it was confirmed that Cp 1 had an excellent efficiency of converting to the anagen phase compared to Minoxidil. Meanwhile, it was shown that 66.0 ± 3.4% of the Cp 1 microneedle administration group (Cp 1conv-DMN) and 72.3 ± 2.5% of the Cp 1 candlelit-shaped microneedle administration group (Cp 1CMN) were converted to the anagen phase, and it was confirmed that the anagen phase conversion effect was significantly increased by the administration method using microneedle administration, particularly, candlelit-shaped microneedles (FIG. 1D).

### 3-6. Changes in skin thickness and hair thickness

In order to compare the degree of the development of skin and hair tissues, the thickness of the H&E-stained skin in Example 1-2 was measured under a microscope, and 30 hairs were collected from each treatment group to compare the thicknesses of the hair.

As a result, a more improved skin thickening effect and hair thickness improvement effect could be observed in the Cp 1-applied experimental group (topical CP 1) compared to the Minoxidil-applied positive control group (topical Minoxidil), and when administering using microneedles, especially candlelit-shaped microneedles, a more significantly improved skin thickening effect and hair thickness improvement effect were confirmed (FIGS. 1E, 1F and 1G).

### 3-7. Analysis of skin tissue development markers

The development of skin tissue, which becomes the surrounding support for hair growth, plays an important role in hair growth. As such, in order to compare the degree of the development of skin tissue in 6 experimental groups of the vehicle-applied control group (topical control), the Minoxidil-applied positive control group (topical minoxidil), the Cp 1application group (topical Cp 1), the candlelit-shaped microneedle control group (CMN control), the Cp 1 microneedle administration group (Cp 1conv-DMN) and the Cp 1candlelit-shaped microneedle administration group (Cp 1 CMN), β-catenin and PCNA, which are biomarkers indicating skin tissue proliferation, the vascular endothelial cell marker MECA 32 showing the development of the vascular network around the mouse hair follicle, and the dermal papilla cell (DP) marker Versican (VCAN), which is generated in the hair follicle anagen phase showing the hair follicle anagen phase inducing effect, were observed by immunohistochemical staining. To this end, as in Example 1-2, the back tissue of the mice was collected on Day 21, then the frozen tissue was sectioned, and the fluorescent material-conjugated antibody was treated on the tissue, and then the fluorescence images were analyzed. The antibodies used were β-catenin (BD Biosciences, San Jose, CA, USA), PCNA (LSBIO, Seattle, WA, USA), VCAN (LSBIO, Seattle, WA, USA) and MECA-32 (Novus Biologicals, CA, USA), respectively.

As a result, β-catenin was shown to be higher when Cp 1was applied or administered with a microneedle than when Minoxidil was applied, and in particular, when Cp 1was administered using a candlelit-shaped microneedle, the highest β-catenin expression effect was shown (FIG. 2A and FIG. 2B). Meanwhile, when using a microneedle, the expression level of PCNA was high regardless of the drug administration (FIGS. 2A and 2C), which is judged to be an effect exerted by the microneedle stimulating the skin.

In the case of MECA 3, it was found that the expression level of MECA 3 increased in the order of Minoxidil application, Cp 1application, Cp 1administration using a general microneedle and M-19 administration using a candlelit-shaped microneedle (FIGS. 0D and 2E), and Cp 1showed a higher vasodilation effect than Minoxidil. In addition, compared to applying Cp 1 the vasodilation effect was further improved when using a microneedle, particularly, a candlelit-shaped microneedle, and it was confirmed that the supply of nutrients through the improvement of blood flow in the hair follicles may be significantly promoted.

In addition, in the case of Versican (VCAN), it was found that the expression level of VCAN increased in the order of Minoxidil application, Cp 1 application, Cp 1 administration using a general microneedle and M-19 administration using a candlelit-shaped microneedle (FIGS. 2F, 2G), and it was confirmed that the anagen phase of hair follicles was promoted in the order of Minoxidil application, Cp 1 application, CP 1 administration using a general microneedle and M-19 administration using a candlelit-shaped microneedle, which indicated that dermal papilla cells could be actively generated.

As described above, specific parts of the content of the present invention are described in detail, and for those of ordinary skill in the art, it will be apparent that these specific descriptions are only preferred exemplary embodiments, and the scope of the present invention is not limited thereby. Accordingly, the substantial scope of the present invention will be defined by the appended claims and their equivalents.

This research was supported by a grant of the Korea Health Technology R&D Project through the Korea Health Industry Development Institute (KHDI), funded by the Ministry of Health & Welfare, Republic of Korea (grant number: HI20C2083).

## Claims

1. A transdermal pharmaceutical composition comprising a compound of formula I or a pharmaceutically acceptable salt thereof 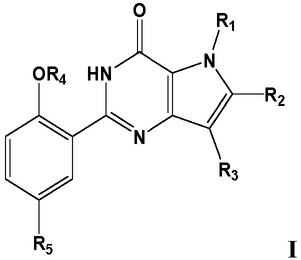 wherein
R₁ is C₁-C₃alkyl, preferably methyl or ethyl, further preferably ethyl;
R₂ is H, CHO or CH=N-OH, preferably H, CHO or (E)-CH=N-OH, further preferably H;
R₃ is C₁-C₄alkyl, preferably ethyl or propyl, further preferably n-propyl;
R₄ is C₁-C₆alkyl, preferably ethyl or propyl, further preferably n-propyl;
R₅ is SO₂NR₆R₇, wherein R₆ and R₇ are together with the nitrogen atom to which they are attached form a heterocyclic ring, wherein said heterocyclic ring is selected from piperidine and piperazine, wherein said heterocyclic ring is substituted with at least one Rs, and wherein said R₈ is independently selected from C₁-C₆alkyl optionally substituted with OH, ONO₂, wherein at least one of said at least one R₈ comprises at least one ONO₂ moiety.

2. The transdermal pharmaceutical composition of claim 1, wherein R₁ is methyl or ethyl; R₂ is H; R₃ is ethyl or n-propyl; and R₄ is ethyl or n-propyl.

3. The transdermal pharmaceutical composition of claim 1, wherein R₁ is ethyl; R₂ is H; R₃ is *n*-propyl; and R₄ is *n*-propyl.

4. The transdermal pharmaceutical composition of claim 1, wherein said compound of formula **I** is a compound selected from the group consisting of:

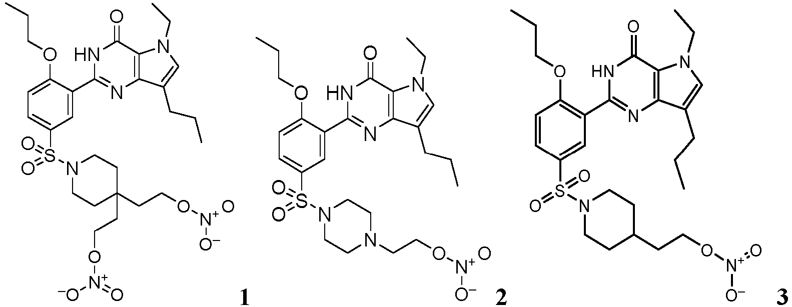

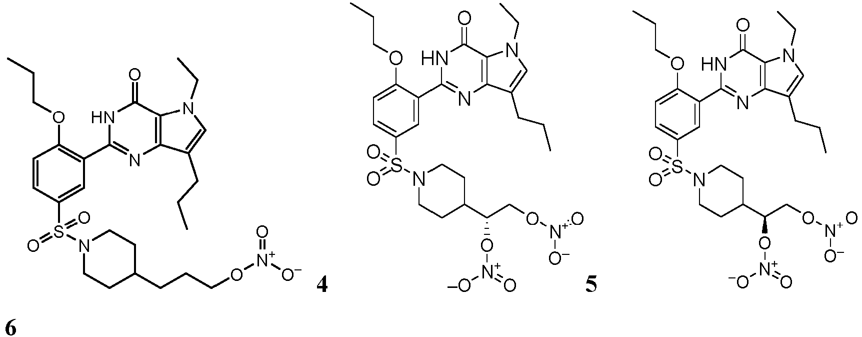

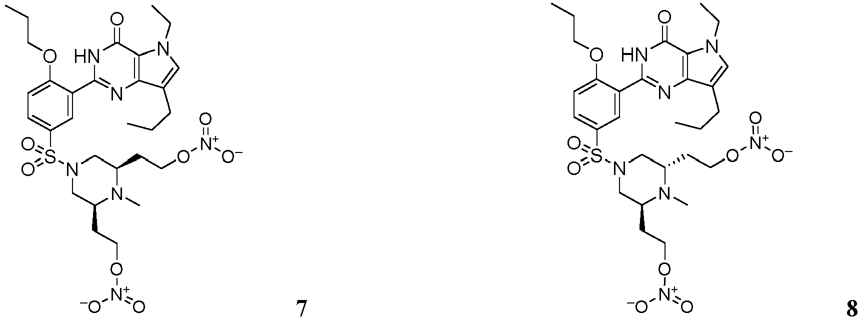

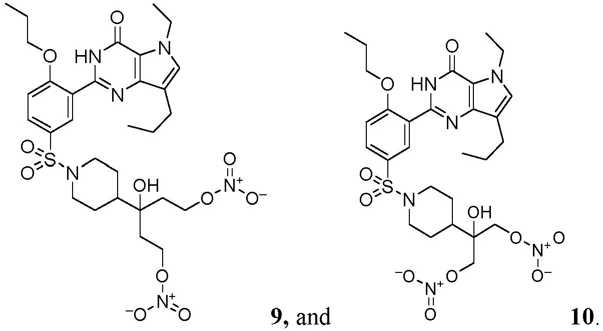

5. The transdermal pharmaceutical composition of claim 1, wherein said compound of formula **I** is a compound selected from the group consisting of:
(1-((3-(5-ethyl-4-oxo-7-propyl-4,5-dihydro-3H-pyrrolo[3,2-d]pyrimidin-2-yl)-4-propoxyphenyl)sulfonyl)piperidine-4,4-diyl)bis(ethane-2,1-diyl) dinitrate **(1);**
(1-((3-(5-ethyl-4-oxo-7-propyl-4,5-dihydro-3H-pyrrolo[3,2-d]pyrimidin-2-yl)-4-propoxyphenyl)sulfonyl)piperidine-4,4-diyl)bis(ethane-2,1-diyl) dinitrate **(1);**
2-(4-((3-(5-ethyl-4-oxo-7-propyl-4,5-dihydro-3H-pyrrolo[3,2-d]pyrimidin-2-yl)-4-propoxyphenyl)sulfonyl)piperazin-1-yl)ethyl nitrate **(2);**
2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethyl nitrate **(3);**
3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)propyl nitrate **(4);**
(R)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate **(5);**
(S)-1-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)ethane-1,2-diyl dinitrate **(6);**
((2R,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate (7);
((2S,6S)-4-((3-(5-ethyl-4-oxo-7-propyl-4,5-dihydro-3H-pyrrolo[3,2-d]pyrimidin-2-yl)-4-propoxyphenyl)sulfonyl)-1-methylpiperazine-2,6-diyl)bis(ethane-2,1-diyl) dinitrate **(8);**
3-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-3-hydroxypentane-1,5-diyl dinitrate **(9);** and
2-(1-((3-(5-ethyl-4-oxo-7-propyl-4,5-dihydro-3H-pyrrolo[3,2-d]pyrimidin-2-yl)-4-propoxyphenyl)sulfonyl)piperidin-4-yl)-2-hydroxypropane-1,3-diyl dinitrate **(10).**

6. The transdermal pharmaceutical of claim 1, wherein said compound of formula **I** is Compound 1 or Compound 2

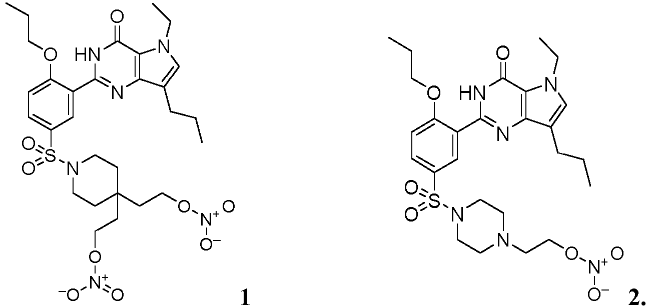

7. The transdermal pharmaceutical composition of claim 1, wherein the composition comprises 0.00005 to 0.5% (w/w) of the compound represented by formula I or the pharmaceutically acceptable salt thereof.

8. The transdermal pharmaceutical composition of claim 1,
wherein the transdermal pharmaceutical composition is for preventing, ameliorating or treating one or more disease or disorders selected from the group consisting of
skin diseases, skin aging, steroid-induced skin atrophy;
erectile dysfunction; and
hair loss or hair growth.

9. The transdermal pharmaceutical composition of claim 8,
wherein the disease or disorder is a hair loss and the transdermal pharmaceutical composition prevents hair loss or promotes hair growth via
(i) inducing anagen of hair follicles;
(ii) promoting melanogenesis;
(iii) increasing the number of hair follicles;
(iv) increasing skin thickness;
(v) promoting angiogenesis;
(vi) proliferating cells in outer root sheath; and/or
(vii) enhancing blood flow in the hair follicle.

10. The transdermal pharmaceutical composition of claim 8, wherein the hair loss is androgenetic alopecia (AGA) or chemotherapy-induced alopecia (CIA) or alopecia areata.

11. A kit comprising:
(i) a first kit component comprising the transdermal pharmaceutical composition of any one of claims 1 to 10; and
(ii) a second kit component comprising an adhesive or non-adhesive patch or device suitable to apply the transdermal pharmaceutical composition to the skin of a subject.

12. A solid composition for preparing the transdermal pharmaceutical composition of any one of claims 1 to 10, wherein the solid composition is dissolvable in liquid solvent, and
wherein the liquid composition formed from the dissolved solid composition comprises 0.00005 to 0.5% (w/w) of a compound of Claim 1 or a pharmaceutically acceptable salt thereof.

13. A microneedle structure on which the composition of any one of claims 1 to 10 is mounted.

14. The microneedle structure of claim 13, wherein the microneedle structure is a microneedle patch or a microneedle applicator.

15. The microneedle structure of claim 13, wherein the microneedle structure comprises a solid microneedle, a coated microneedle, a dissolving microneedle, a hollow microneedle or a candlelit-shaped microneedle.

16. The microneedle structure of claim 15, wherein the microneedle structure comprises a biocompatible polymer selected from the group consisting of hyaluronic acid, collagen, polyvinylpyrrolidone, polylactic acid, polylactic glycolic acid, polylactide, polyglycolic acid, polyethylene glycol, polycaprolactone, poly-amino acid, polypropylene fumarate, poly-gamma glutamic acid, polyvinyl alcohol, polyethyleneimine, albumin, dextran, fibrin, elastin, gelatin, alginic acid, polyacrylic acid, carboxymethylcellulose, and mixtures or copolymers thereof.

17. The microneedle structure of claim 13, wherein the composition is injected once within 2 to 15 days using the microneedle structure.
